# EUROPEAN PATENT APPLICATION

(11) **EP 2 805 608 A1**
(43) Date of publication of application: **26.11.2014**
(21) Application number: 11875750.9
(22) Date of filing: 14.11.2011
(51) Int. Cl.: A01K 1/03

(54) **CONTAINER FOR RAISING SMALL ANIMALS**

(71) Applicant: Kabushiki Kaisha Tominaga Jyushi Kogyosho, Higashiosaka-shi Osaka 577-0025 (JP)
(72) Inventor: TOMINAGA Kazutoshi, Higashiosaka-shi Osaka 577-0025 (JP)
(74) Representative: Viering, Jentschura & Partner Patent- und Rechtsanwälte
(86) International application number: PCT/JP2011/076184
(87) International publication number: WO 2013/072984

(57) **Abstract**

The present invention provides a container for raising small animals, which can be manufactured easily and the packaged size of which can also be rendered small. The container for raising small animals includes peripheral walls 21 to 23 enclosing the periphery, and is provided with a hard synthetic resin base 2 having an opened upper end and a top cover 6 which is positioned on the base to cover an upper space of the base 2. The base 2 is formed by assembling at least two independent components.

## Description

### TECHNICAL FIELD

The present invention relates to a container for raising small animals and its base for appreciation breeding of small animals including, e.g., mammals such as rabbits, ferrets, hamsters, and mice, birds such as small birds, reptiles such as tortoises, lizards, and snakes, amphibians such as frogs, insects such as beetles, and stag beetles.

### TECHNICAL BACKGROUND

Patent Documents 1 and 2 disclose containers for raising small animals for appreciation breeding of small animals as mentioned above.

The container for raising small animals is equipped with an upwardly opened base to be arranged at a lower portion, a tray to be accommodated in the base in a drawable manner, a drainboard to be arranged at the upper end portion of the base, and a downwardly opened top cover to be arranged on the base.

In the container for raising small animals, unnecessary objects, such as, e.g., feces and urine excreted by small animals, falling hairs, and spilt foods, fall and are accommodated in the tray. At the time of cleaning, the tray is pulled out from the base and the unnecessary objects accommodated in the tray are disposed.

### PRIOR ART DOCUMENT

### PATENT DOCUMENT

Patent Document 1: Japanese Patent No. 3910602
Patent Document 2: U.S. Patent No. 7380520 Specification

### SUMMARY OF THE INVENTION

### PROBLEMS TO BE SOLVED BY THE INVENTION

In the conventional container for raising small animals, the base is required to form a support portion for supporting the top cover and the drainboard on the inner peripheral surface of the base, and/or a guide portion so that unnecessary objects can be assuredly accommodated inside the tray. Furthermore, the base is also required to form a support mechanism for slidably supporting the tray. In this way, the base becomes complex in shape since it is necessary to form many complex portions.

On the other hand, in many cases, the base for a container for raising small animals is constituted by an integrally molded article made by plastic molding. As explained above, the base is complex in shape, and a die for forming the base also becomes complex in structure. Therefore, the manufacturing of the base becomes difficult, which in turn makes it difficult to manufacture the container for raising small animals. Furthermore, since the base is relatively large in size, the die becomes also large. In terms of this point, the manufacturing of the base is difficult.

Also, since the base is large in size, when packing the container for raising small animals, the size of the packed product becomes large. Therefore, it becomes difficult to carry the container for raising small animals in a packed state, for example.

The preferred embodiments of the present invention were made in view of the aforementioned and/or other problems in the technical background. The preferred embodiments of the present invention can significantly improve upon the existing methods and/or devices.

The present invention was made in view of the aforementioned problems and aims to provide a container for raising small animals and its base capable of being easily manufactured and packed into a small size.

The other objects and advantages of the present invention will be apparent from the following preferred embodiments.

### MEANS TO SOLVE THE PROBLEMS

The present invention provides the following means to solve the aforementioned problems.
[1] A container for raising small animals, comprising:
   a hard synthetic resin base including a peripheral wall enclosing a periphery of the base, an upper end of the base being opened; and
   a top cover configured to be arranged on the base to cover an upper space of the base,
      wherein the base is formed by assembling at least two independent components.
[2] The container for raising small animals as recited in the aforementioned Item 1, wherein the peripheral wall of the base includes four walls including a front wall, a rear wall and side walls, the walls constituting the independent components.
[3] The container for raising small animals as recited in the aforementioned Item 2, wherein a tray configured to receive unnecessary obj ects is accommodated in the base in a drawable manner, and a front opening for taking in and out the tray with respect to the base is provided at a lower side of the front wall.
[4] The container for raising small animals as recited in the aforementioned Item 3, wherein a through-hole is formed in portions of the rear wall and both the side walls corresponding to the tray.
[5] The container for raising small animals as recited in the aforementioned Item 3 or 4, wherein a material of the tray is a paper or a plastic film.
[6] The container for raising small animals as recited in any one of the aforementioned Items 3 to 5, wherein the independent component is formed to have a size capable of being accommodated in the tray.
[7] A base for a container for raising small animals to which an upper cover having an opened lower end is attached, comprising:
   a hard synthetic resin peripheral wall including a front wall, a rear wall and side walls and having an opened upper end,
      wherein the front wall, the rear wall and the side walls are each constituted by an independent component, and the peripheral wall is formed by assembling the independent components.

### EFFECTS OF THE INVENTION

According to the container for raising small animals of the invention [1], the base is constituted by two or more independent components, so the base can be manufactured every independent components to be manufactured. Since the structure of each independent component becomes simple in comparison to the overall structure of the base, each component can be manufactured easily. Therefore, the base can be easily manufactured, which in turn makes it possible to easily manufacture the container for raising small animals.

Furthermore, in the container for raising small animals of the present invention, by packing the independent components so as to be overlapped, it is also possible to make the packing size small as well.

According to the container for raising small animals of the invention [2], the peripheral four walls are each constituted by two or more independent components. Each wall is formed into a shape approximate to a two-dimensional shape, thereby making the structure even simpler. Therefore, the container for raising small animals can be manufactured even more easily and packed into more compact size.

According to the container for raising small animals of the invention [3], the unnecessary objects in the container can be easily disposed by simply pulling out the tray and disposing the unnecessary objects in the tray.

According to the container for raising small animals of the invention [4], since a through-hole is formed in each of the rear wall and both side walls, the weight and the material consumption can be reduced for the amount of the holes.

According to the container for raising small animals of the invention [5], the tray can be disposable. Therefore, when disposing unnecessary objects accumulated in the tray, the unnecessary objects can be disposed together with the tray. Therefore, the disposal of unnecessary objects can be easily executed.

According to the container for raising small animals of the invention [6], since each independent component can be accommodated in the tray, the container can be packed in an even more compact manner.

According to the base for the container for raising small animals of the invention [7], in the same manner as mentioned above, the base can be easily manufactured and packed in a compact manner.

### BRIEF DESCRIPTION OF THE DRAWINGS

[Fig. 1] Fig. 1 is a perspective view showing a container for raising small animals according to an embodiment of the present invention.
[Fig. 2] Fig. 2 is a perspective view showing the container for raising small animals of the embodiment in a state in which the top cover is detached.
[Fig. 3] Fig. 3 is a perspective view showing the state in which the base is pulled out from the tray in the container for raising small animals of the embodiment.
[Fig. 4] Fig. 4 is a perspective view showing the base of the container for raising small animals of the embodiment in an exploded state.
[Fig. 5] Fig. 5 is a cross-sectional view taken along the line V-V in Fig. 2.
[Fig. 6] Fig. 6 is a cross-sectional view taken along the line VI-VI line in Fig. 2.
[Fig. 7] Fig. 7 is a schematic view showing the state in which the components of the disassembled base are accommodated in the tray in the container for raising small animals of the embodiment.

### EMBODIMENTS FOR CARRYING OUT THE INVENTION

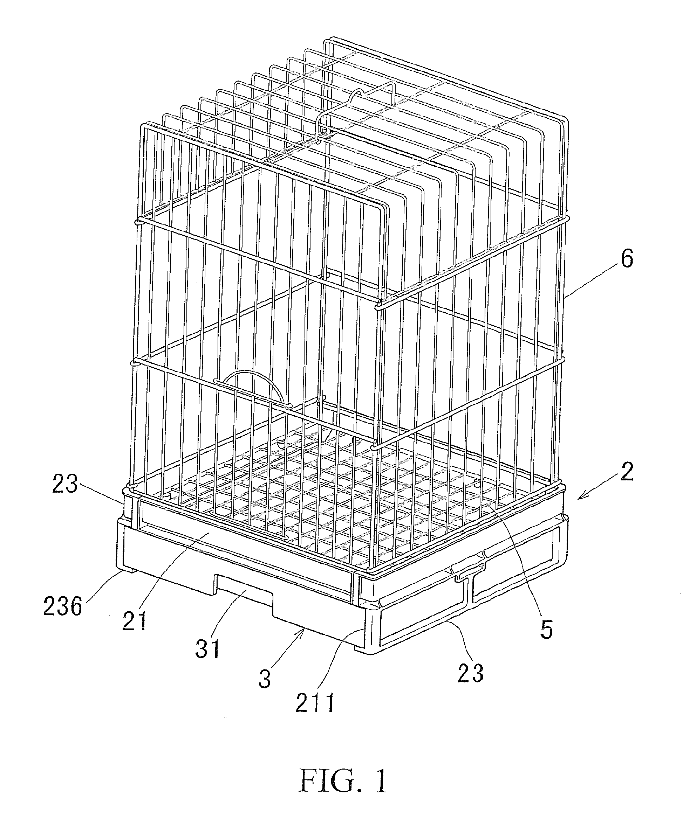

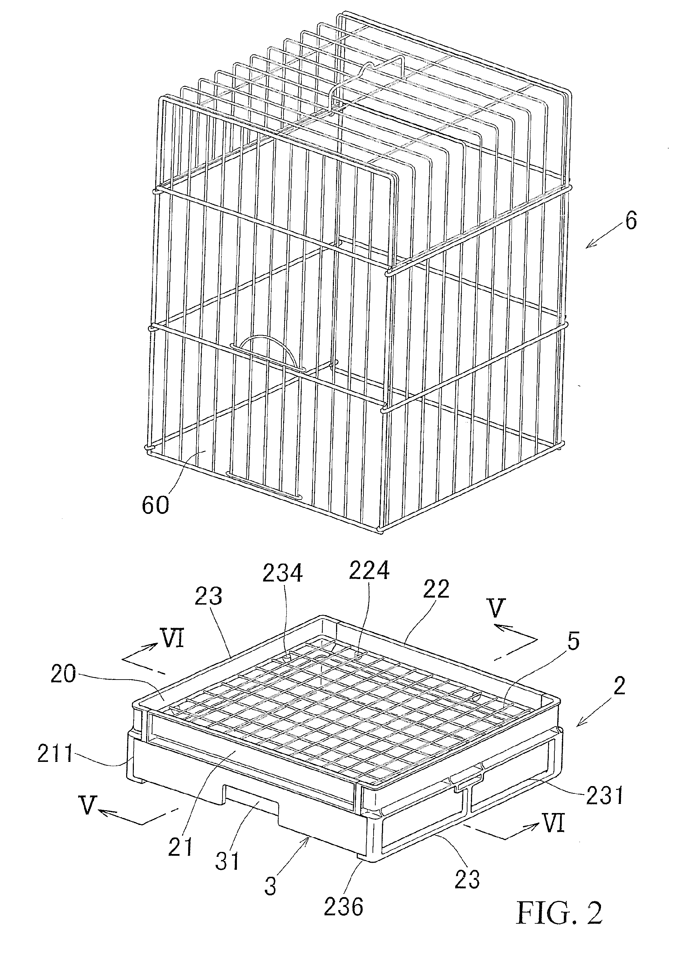

Fig. 1 is a perspective view showing a container for raising small animals of an embodiment of the present invention, and Fig. 2 is a perspective view showing the container for raising small animals in a state in which the top cover is removed.

As shown in both the figures, the container for raising small animals of this embodiment can be preferably used as a bird cage. The container for raising small animals is equipped with a base 2 to be arranged at a bottom portion, a tray 3 to be accommodated in the base 2 in a drawable manner, a drainboard 5 to be arranged in the base 2, and a top cover 6 to be arranged on the base 2 so as to cover the upper space of the base.

The base 2 is formed into an approximately rectangular shape in plan view. The base 2 is equipped with a front wall 21 to be arranged on a front side, a rear wall 22 to be arranged on a rear side, and both side walls 23 and 23 to be arranged on both left and right sides. In this embodiment, the peripheral wall is constituted by the front wall 21, the rear wall 22 and both the side walls 23 and 23.

The base 2 has an opening 20 at its upper end. The base 2 opens upward via the opening 20. Furthermore, the base 2 opens downward.

In this embodiment, the front wall 21, the rear wall 22, and both the side walls 23 and 23 are each constituted by an independent component. Also, the base 2 is formed by assembling each of the walls 21 to 23 as components.

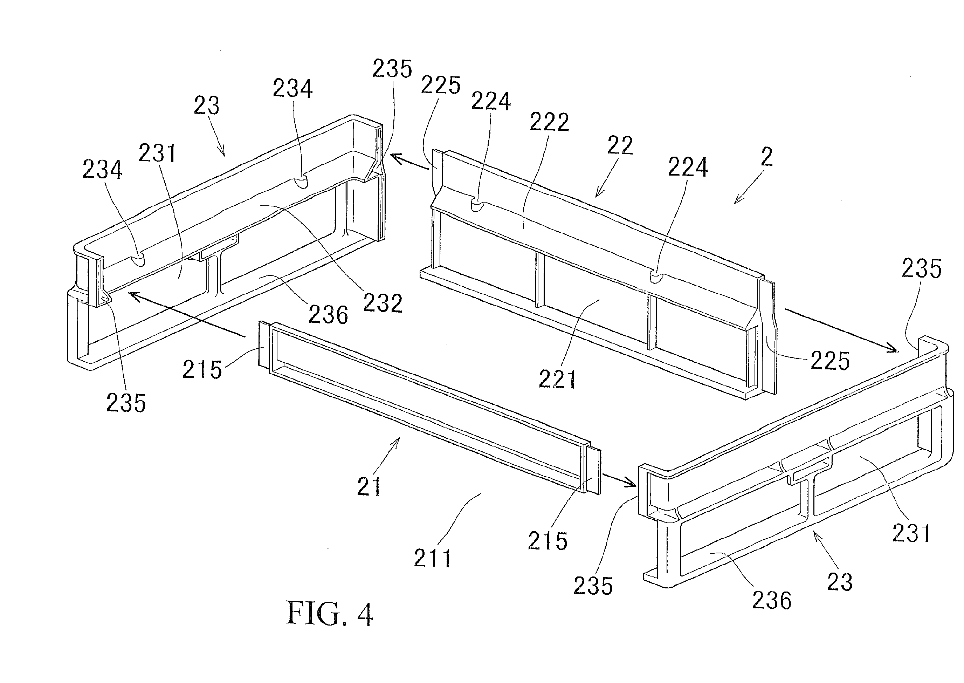

That is, as shown in Fig. 4, each of the walls 21 to 23 is constituted by a hard synthetic resin integrally molded article.

On both side end portions of the front wall 21, insertion pieces 215 and 215 are formed so as to protrude sideways. Furthermore, insertion pieces 225 and 225 protruding sideways are formed on both side end portions of the rear wall 22. Also, insertion holes 235 and 235 corresponding to the insertion pieces 215 and 225 of the front wall 21 and the rear wall 22 are formed in front and rear end portions of both side walls 23 and 23.

Then, the insertion pieces 215 and 225 are inserted into and fixed to the insertion holes 235 and 235. In this way, as shown in Figs. 1 to 3, the walls 21 to 23 are assembled with each other to form the base 2.

In this embodiment, when inserted into the insertion hole 235 and 235, the insertion pieces 215 and 225 are configured to be retained in the insertion holes 235 and 235. Therefore, when the insertion pieces 215 and 225 are inserted into the insertion holes 235 and 235, the walls 21 to 23 are fixed with each other, thereby preventing each of the walls 21 to 23 to be easily separated.

In the present invention, the means for retaining the insertion pieces 215 and 225 in the insertion holes 235 and 235 can be any known means. For example, it can be configured such that a retaining protrusion is formed on each of the insertion pieces 215 and 225, and a protrusion receiving portion is formed in each of the insertion holes 235 and 235 so that the retaining protrusions can be elastically engaged with the protrusion receiving portions when the insertion pieces 215 and 225 are inserted into the insertion holes 235 and 235. In this way, the insertion pieces 215 and 225 are retained in the insertion holes 235 and 235.

Furthermore, in the present invention, the insertion pieces 215 and 225 can be fixed in a state in which the insertion pieces are inserted into the insertion holes 235 and 235 using an adhesive agent.

The bottom portion of each of the rear wall 22 and both side walls 23 of the base 2 is formed into a frame shape. The inside of the frame of each of the rear wall 22 and both side walls 23 is structured as a through-hole 221 and 231. The inside portion of the base 2 opens rearward and sideways via the through-holes 221 and 231.

Also, the bottom portion of the front wall 21 of the base 2 does not exist and is open. In this way, in a state in which the walls 21 to 23 are assembled, a front opening 211 is formed at a lower portion of the front wall 21 of the base 2. The inside of the base 2 is open frontward via the front opening 211.

At the lower end edges of both side walls 23 and 23, tray receiving portions 236 and 236 are integrally formed so as to project inward. The tray receiving portions 236 and 236 are formed continuously along the front and rear direction.

In this embodiment, the tray receiving portions 236 and 236 are continuously formed, but not limited to that. In this present invention, the tray receiving portions can be partially formed.

The tray 3 is formed into a flat box shape having an opening 20 at the upper end. The tray 3 opens upward via the upper end opening 30. The front wall of the tray 3 is constituted by double walls. The front side wall of the double walls is formed so that the lower side of the central portion is cut out. The cutout portion constitutes a handle 31.

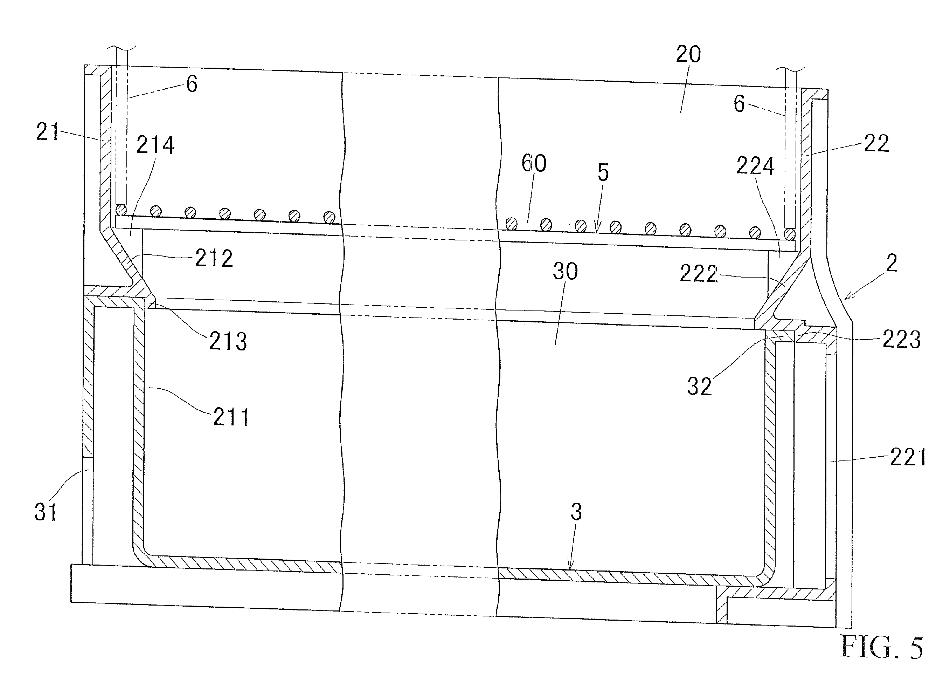

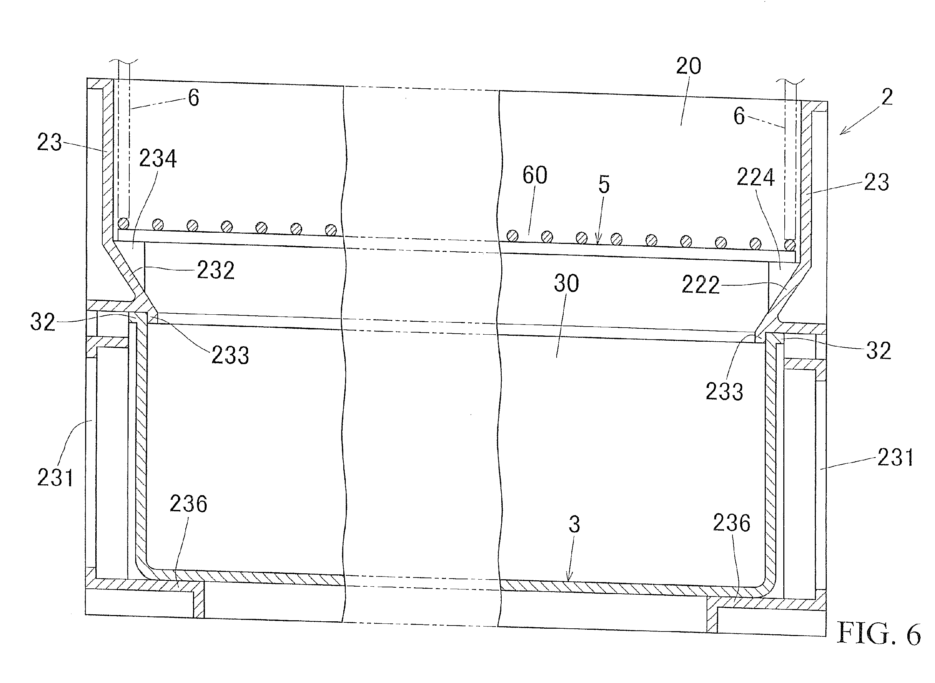

Also, ribs 32 are formed in an outwardly protruded manner on the upper end edges of the rear wall and both side walls of the tray 3 (see Figs. 5 and 6.) The ribs 32 are formed on the entire circumference of the upper end outer peripheral edge portion of the tray 3 with the exception of the portion of the front wall of the tray 3.

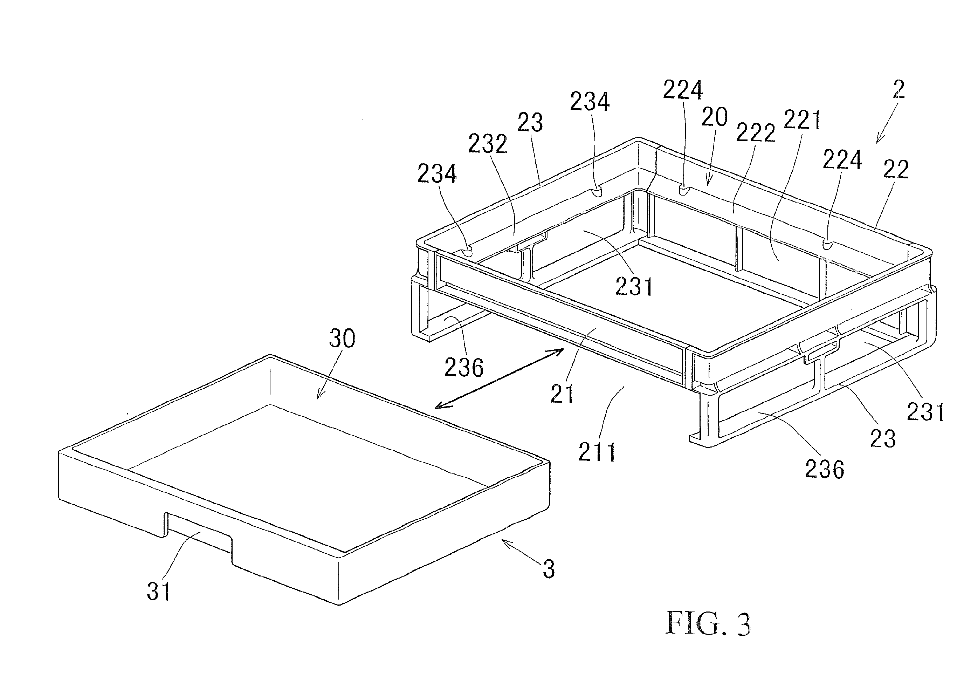

As shown in Fig. 3, the tray 3 is formed so that the front cross-sectional shape corresponds to the shape of the front opening 211 of the base 2. Therefore, the tray 3 can be accommodated in the base 2 from the front opening 211 of the base 2. In this accommodated state, both side edges of the bottom surface of the tray 3 are supported by the tray receiving portions 236 and 236 of the base 2, and the tray 3 is thereby held in the base 2. Also, when the tray 3 in the accommodated state is pulled forward, the tray 3 can be pulled out to the front of the base 2.

When the tray 3 is pulled in and out from the base 2, the operation to pull the tray 3 in and out can be executed easily by utilizing the handle 31 of the tray 3.

During the operation to pull the tray 3 in and out, both side edges of the bottom surface of the tray 3 slides on the tray receiving portions 236 and 236. Therefore, the tray receiving portions 236 and 236 function as guide members for the tray 3.

As shown in Fig. 5, the guide walls 212 and 222 are integrally formed on each of inner surfaces of the front wall 21 and the rear wall 22 of the base 2. Each guide wall 212 and 222 is inclined so as to be positioned gradually inward toward the lower portion. The lower end position of each guide wall 212 and 222 is arranged at an upper inner side with respect to the front wall and the rear wall of the accommodated tray 3. Also, the inner side surface of each guide wall 212 and 222 is an inclined surface inclining downward and inward. Therefore, the inner side surface of each guide wall 212 and 222 functions as a guiding surface for guiding unnecessary objects such as feces and urine excreted by animals, falling hairs, and spilt foods into the tray 3, as explained below.

At the lower end of the guide wall 212 of the front wall 21, a positioning portion 213 is integrally formed so as to protrude downward. Furthermore, on the lower side of the guide wall 222 of the rear wall 22, a positioning portion 223 is formed. These positioning portions 213 and 223 are each continuously formed along the right and left direction.

By these positioning portions 213 and 223, the positioning of the tray 3 is made when the tray 3 is accommodated in the base 2. That is, when the tray 3 is completely accommodated in the base 2, the upper end of the inner surface of the front wall of the tray 3 abuts against the positioning portion 213 and the upper end of the outer surface of the rear wall of the tray 3 abuts against the positioning portion 223. In this way, the front wall and the rear wall of the tray 3 are positioned and the positioning of the tray 3 in the base 2 in the front rear direction is made.

In addition, the rear wall of the tray 3 is formed lower than the front wall. Therefore, when the tray 3 is pulled in and out of the base 2 via the front opening 211, the rear wall of the tray 3 is prevented from interfering with the positioning portion 213 of the front wall 21 of the base 2.

As shown in Fig. 6, guide walls 232 and 232 are integrally formed on inner side surfaces of both side walls 23 and 23 of the base 2. The guide walls 232 and 232 are inclined so as to be positioned gradually inward toward the lower portion. The inner side surface of each guide wall 232 and 232 is an inclined surface inclining downward and inward. The inner side surface of the guide wall 232 and 232 functions as a guiding surface for guiding unnecessary objects into the tray 3.

At the lower ends of the guide walls 232 and 232 of both side walls 23 and 23, guide pieces 233 and 233 are integrally formed so as to protrude downward. The guide pieces 233 and 233 are continuously formed along the front and rear direction.

The guide pieces 233 and 233 come into contact with the inner side surface of both side walls of the tray 3 when the tray 3 is pulled in and out of the base 2, and the tray 3 is thereby guided in the front and rear direction. In this manner, the tray 3 can be accurately slid along the front and rear direction, thereby making it possible to smoothly execute the operation to pull the tray 3 in and out. Furthermore, the guide pieces 233 and 233 come into contact with the inner surfaces of both side walls of the tray 3, and the positioning of the tray 3 in the base 2 in the right and left direction is made.

A plurality of drainboard supporting protrusions 214 and 224 are integrally formed on the upper portion of the guide walls 212 and 222 on the inner side surfaces of the front wall 21 and the rear wall 22 of the base 2 at intervals in the right and left direction. Furthermore, a plurality of drainboard supporting protrusions 234 and 234 are integrally formed on the upper portions of the guide walls 232 and 232 on the inner side surfaces of both side walls 23 and 23 at intervals in the front and rear direction.

In addition, among both left and right side walls 23 and 23, one of the side walls 23 is an enantiomer to the other of the side walls 23, and one of the side walls 23 and the other of the side walls 23 essentially have the same structure.

As shown in Fig. 2, etc. , the drainboard 5 is constituted by a number of linear members arranged in a lattice shape or a reticular shape.

In plan view, the outer peripheral shape of the drainboard 5 is formed so as to correspond to the inner peripheral shape of the upper end portion of the base 2. Therefore, the drainboard 5 can be accommodated in the base 2 from the upper end opening in a fitted state. Furthermore, in the accommodated state, as shown in Figs. 5 and 6, the outer peripheral edge portion of the drainboard 5 is supported by the drainboard supporting protrusions 214, 224 and 234. In this way, the drainboard 5 is held in a horizontal state at the upper portion of the guide walls 212, 222, and 232 in the base 2.

In this embodiment, the drainboard 5 is constituted by a number linear members assembled into a lattice or reticular shape, but the present invention is not limited to that. In the present invention, the drainboard can be formed so that a plurality of band plate members are arranged in parallel with each other while forming a gap therebetween. Furthermore, the drainboard can be formed by a plate-like member having a number of holes and/or slits.

As shown in Figs. 1 and 2, the top cover 6 has a box shape having an opening 60 at the lower end. The top cover 6 opens downward via the opening 60 at the lower end. The top wall and the four peripheral side walls of the top cover 6 are constituted by a number of linear members arranged and fixed in a lattice shape or palisade shape. In the top cover 6, the four peripheral side walls and the top wall are each constituted to be detachable.

In plan view, the outer peripheral shape of the peripheral edge portion of the lower end opening 60 of the top cover 6 is formed so as to correspond to the inner peripheral shape of the peripheral edge portion of the upper end opening 20 of the base 2. Therefore, the lower end opening edge portion of the top cover 6 can be accommodated inside the base 2 from the upper end opening 20. Furthermore, in the accommodated state, the lower end opening edge portion of the top cover 6 is supported by the drainboard supporting protrusions 214, 224 and 234 via the outer peripheral edge portions of the drainboard 5. In this way, the top cover 6 is held on the base 2 and the upper space of the base 2 is covered by the top cover 6.

In this embodiment, a cage type and a basket type top cover 6 made by a number of linear members are used, but the present invention is not limited to that. For example, a clear plastic top cover having an open lower end can be used. In that case, to secure breathability, a plurality of venting holes and/or slits can be formed in the top cover.

Furthermore, when using a plastic top cover, the four peripheral side walls and the top wall can be structured so as to be detachable with each other, or the entire top cover can be integrally formed.

In this embodiment, the lower end opening edge portion of the top cover 3 is supported by the drainboard supporting protrusions 214, 224, and 234b via the outer peripheral edge portion of the drainboard 5, but the present invention is not limited to that. For example, it can be configured such that a gap is formed between the outer peripheral edge portion of the drainboard 5 and the four peripheral side walls 21 to 23 of the base 2, the lower end opening edge portion of the top cover 3 is arranged in the gap so that the lower end opening edge portion of the top cover 3 is supported directly by the drainboard supporting protrusion 214, 224 and 234.

According to the container for raising small animals of this embodiment having aforementioned structure, since it is equipped with the drawable tray 3 in the base 2, unnecessary objects generated in the container, such as feces and urine excreted by animals, falling hair, and spilt foods, can pass through the drainboard 5 and be received in the tray 3. Therefore, unnecessary objects can be easily disposed only by pulling out the tray 3 forward from the base 2 and disposing the unnecessary objects inside the tray 3.

In this embodiment, a disposable tray 3 can also be used. That is, the tray 3 can be constituted by comparatively inexpensive and soft members. For example, a tray 3 formed by paper can be used, or a tray 3 formed by plastic films and/or sheets formed by pneumatic forming, such as vacuum molding, can be used. The disposable tray 3 is accommodated in the base 2. Then, when disposing the unnecessary objects accumulated in the tray 3, the tray 3 is pulled out from the base 2 and the unnecessary objects are disposed together with the tray 3. At the time of disposal, for example, the tray 3 is rolled up so as to wrap the unnecessary objects with the tray 3, and the unnecessary objects are disposed together with the tray 3 in that state. In that way, inconveniences such as the unnecessary objects scattering to the periphery can be prevented.

Furthermore, when using the disposable tray 3, after disposing the unnecessary objects, there is no need to clean the tray 3 to reuse the tray 3.

In addition, after disposing the tray 3 with the unnecessary objects, a new tray 3 can be arranged to the base 2.

On the inner peripheral side surface of the base 2, the guide walls 212, 222, and 232 extending further inside than the peripheral wall of the tray 3 are formed. Therefore, the unnecessary objects falling in the inner peripheral edge of the base 2 are guided by the inner side surface (guiding surface) of the guide walls 212, 222, and 232 and assuredly accommodated in the tray 3. Therefore, an inconvenience that unnecessary objects get into the gap between the base 2 and the tray 3 can be assuredly prevented.

Also, in this embodiment, the base 2 is constituted by the front wall 21, the rear wall 22, and both side walls 23 and 23, which are four independent components, and each of the walls 21 to 23 can be detached. In other words, the container for raising small animals of this embodiment is equipped with a knockdown type base 2. Therefore, the base 2 can be easily manufactured, which in turn can easily manufacture the container for raising small animals.

That is, for the base 2, it is required to form a plurality of complex members, such as the guide walls 212, 222 and 232, the drainboard supporting protrusion 214, 224 and 234, and the tray receiving portion 236. Therefore, if the entire base 2 is an integrally molded article, the shape of the base 2 becomes extremely complex. Therefore, if it is attempted to form the base 2 having such a complex shape by resin heat molding, the structure of the molding die for forming the base becomes complex. As a result, the manufacturing of the die becomes difficult and there is a risk that the manufacturing of the container for raising small animals becomes difficult. In addition, since the size of the overall base 2 is large, the molding die for forming the base is large. Therefore, the manufacturing of the die becomes even more difficult in terms of the aforementioned point as well, and the manufacturing of the base may become even more difficult.

Therefore, in this embodiment, since the base 2 is constituted by four detachable walls 21 to 23, the base 2 can be manufactured each wall 21 to 23 separately. The structure of each of the walls 21 to 23 is simple in comparison to the overall structure of the base. Therefore, the structure of the die for forming each wall 21 to 23 becomes simple. Therefore, the die can be easily manufactured with excellent accuracy, and the base 2 can be easily manufactured with excellent accuracy, which in turn can easily manufacture the container for raising small animals with excellent accuracy. Furthermore, the costs can be reduced.

Further, when compared to the overall base, each of the walls 21 to 23 is small in size, so the die for forming each wall is also small. For the aforementioned reasons, the die can be more easily manufactured with excellent accuracy and the base 2 and the container for raising small animals can be even more easily manufactured with excellent accuracy. Furthermore, the costs can be reduced even further.

Also, in the container for raising small animals of this embodiment, the four walls 21 to 23 of the base 2 are detachable, so when packing, the size of the packed product can be made small. That is, by arranging the four walls 21 to 23 in a detached state in an overlapped manner, it can be made compact. Therefore, by packing in a state in which each wall 21 to 23 is overlapped in a compact manner, the size of the packed product can be made small. Therefore, when displaying or storing the container for raising small animals of this embodiment as a product, it can be arranged in an efficient manner in terms of space. Furthermore, when carrying the container for raising small animals of this embodiment as a product, it can be easily carried since it is not bulky.

Especially in this embodiment, each of the front wall 21, the rear wall 22, and both side walls 23 and 23 is constituted as an independent component. Since each wall 21 to 23 has a shape approximate to a plate-like two-dimensional shape, the structure can be made even simpler. Therefore, the container for raising small animals of this embodiment can be manufactured even more easily and packed in an even more compact manner.

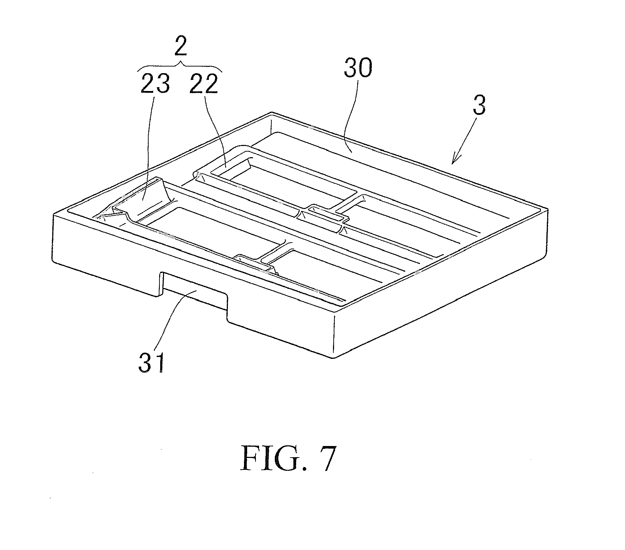

As shown in Fig. 7, in this embodiment, each of the walls 21 to 23 in a detached state is formed to be a size capable of being accommodated in the tray 3. Therefore, by accommodating each of the walls 21 to 23 in a detached state in the tray 3, the container for raising small animals of this embodiment can be packed to be even more compact. Furthermore, in the container for raising small animals of this embodiment, if the top cover 6 is constituted so that each constitutional member can be accommodated in the tray 3 when the top cover 6 is disintegrated into each wall portion, it can be packed more assuredly in a compact manner.

Also, in this embodiment, the lower side portion of the rear wall 22 and both side walls 23 and 23 are each formed into a frame shape and the inside of the frame constitutes the through-hole 221 and 231. Therefore, the base 2 of this embodiment can be made light in comparison to when the through-holes 221 and 231 are not formed. Furthermore, the resin material constituting the rear wall 22 and both side walls 23 and 23 can be reduced, thereby making it possible to reduce the cost as well.

The aforementioned embodiment was explained by exemplifying a case in which the base 2 is constituted by four components, i.e., the front wall 21, the rear wall 22, and both side walls 23 and 23, but the present invention is not limited to that. For example, the present invention can be constituted by two independent components, three independent components, or five or more independent components.

Furthermore, in the aforementioned embodiment, it is configured such that the base 2 can be divided in the circumferential direction, but this present invention is not limited to that. For example, in the present invention, it can be configured such that each of the walls 21 to 23 of the base 2 can be divided in the up and down direction.

In the present invention, the base can be formed by assembling at least two or more independent components.

Also, the aforementioned embodiment was explained by exemplifying a case in which the container for raising small animals is used as a bird cage, but the present invention is not limited to that. The present invention can be used as a container for appreciation breeding of small animals including, e.g., mammals such as rabbits, ferrets, hamsters, and mice, birds such as a small bird, reptiles such as tortoises, lizards, and snakes, amphibians such as frogs, insects such as beetles, and stag beetles.

The terms and descriptions used herein are used only for explanatory purposes and the present invention is not limited to them. The present invention allows various design-changes falling within the claimed scope of the present invention unless it deviates from the spirits of the invention.

While the present invention may be embodied in many different forms, a number of illustrative embodiments are described herein with the understanding that the present disclosure is to be considered as providing examples of the principles of the invention and such examples are not intended to limit the invention to preferred embodiments described herein and/or illustrated herein.

While illustrative embodiments of the invention have been described herein, the present invention is not limited to the various preferred embodiments described herein, but includes any and all embodiments having equivalent elements, modifications, omissions, combinations (e.g., of aspects across various embodiments), adaptations and/or alterations as would be appreciated by those in the art based on the present disclosure. The limitations in the claims are to be interpreted broadly based on the language employed in the claims and not limited to examples described in the present specification or during the prosecution of the application, which examples are to be construed as non-exclusive.

### INDUSTRIAL APPLICABILITY

The container for raising small animals of the present invention can be used for appreciation breeding of small animals.

### EXPLANATION OF SYMBOLS

- 2:: base
- 21:: front wall
- 211:: front opening
- 22:: rear wall
- 221:: through-hole
- 23:: side wall
- 231:: through-hole
- 3:: tray
- 6:: top cover

## Claims

1. A container for raising small animals, comprising:
a hard synthetic resin base including a peripheral wall enclosing a periphery of the base, an upper end of the base being opened; and
a top cover configured to be arranged on the base to cover an upper space of the base,
wherein the base is formed by assembling at least two independent components.

2. The container for raising small animals as recited in claim 1, wherein the peripheral wall of the base includes four walls including a front wall, a rear wall and side walls, the walls constituting the independent components.

3. The container for raising small animals as recited in claim 2, wherein a tray configured to receive unnecessary objects is accommodated in the base in a drawable manner, and a front opening for taking in and out the tray with respect to the base is provided at a lower side of the front wall.

4. The container for raising small animals as recited in claim 3, wherein a through-hole is formed in portions of the rear wall and both the side walls corresponding to the tray.

5. The container for raising small animals as recited in claim 3 or 4, wherein a material of the tray is a paper or a plastic film.

6. The container for raising small animals as recited in any one of claims 3 to 5, wherein the independent component is formed to have a size capable of being accommodated in the tray.

7. Abase for a container for raising small animals to which an upper cover having an opened lower end is attached, comprising:
a hard synthetic resin peripheral wall including a front wall, a rear wall and side walls and having an opened upper end,
wherein the front wall, the rear wall and the side walls are each constituted by an independent component, and the peripheral wall is formed by assembling the independent components.

## Amended claims

### Amended claims under Art. 19.1 PCT

**1.** A container for raising small animals, comprising:
a hard synthetic resin base including a peripheral wall enclosing a periphery of the base, an upper end of the base being opened; and
a top cover configured to be arranged on the base to cover an upper space of the base,
wherein the base is formed by assembling at least two independent components.

**2.** The container for raising small animals as recited in claim 1, wherein the peripheral wall of the base includes four walls including a front wall, a rear wall and side walls, the walls constituting the independent components.

**3.** The container for raising small animals as recited in claim 2, wherein a tray for receiving unnecessary objects is accommodated in the base in a drawable manner, and a front opening for taking in and out the tray with respect to the base is provided at a lower side of the front wall.

**4.** The container for raising small animals as recited in claim 3, wherein a through-hole is formed in portions of the rear wall and both the side walls corresponding to the tray.

**5.** The container for raising small animals as recited in claim 3 or 4, wherein a material of the tray is a paper or a plastic film.

**6.** The container for raising small animals as recited in any one of claims 3 to 5, wherein the independent component is formed to have a size capable of being accommodated in the tray.

**7.** (Amended) The container for raising small animals as recited in any one of claims 3 to 6, wherein the tray is a disposable tray.

**8.** (Added) A base for a container for raising small animals to which an upper cover having an opened lower end is attached, comprising:
a hard synthetic resin peripheral wall including a front wall, a rear wall and side walls and having an opened upper end,
wherein the front wall, the rear wall and the side walls are each constituted by an independent component, and the peripheral wall is formed by assembling the independent components.
